# EUROPÄISCHE PATENTSCHRIFT

(11) **EP 3 310 285 B1**
(45) Veröffentlichungstag und Bekanntmachung des Hinweises auf die Patenterteilung: **02.04.2025**
(21) Anmeldenummer: 16787453.6
(22) Anmeldetag: 26.10.2016
(51) Int. Cl.: A61B 18/14

(54) **HOCHFREQUENZWERKZEUG FÜR MEDIZINISCHE RESEKTOSKOPE**
HIGH-FREQUENCY TOOL FOR MEDICAL RESECTOSCOPES
OUTIL À HAUTE FRÉQUENCE POUR RÉSECTOSCOPE MÉDICAL

(30) Priorität: 03.11.2015 DE 102015014088
(43) Veröffentlichungstag der Anmeldung: 25.04.2018
(73) Patentinhaber: Olympus Winter & Ibe GmbH, 22045 Hamburg (DE)
(72) Erfinder: BROCKMANN, Christian, 21279 Hollenstedt (DE); KAISER, Andreas, 22089 Hamburg (DE)
(74) Vertreter: Hoener, Matthias
(86) Internationale Anmeldenummer: PCT/EP2016/075856
(87) Internationale Veröffentlichungsnummer: WO 2017/076721

(56) Entgegenhaltungen:
- DE-A1- 102005 042 312
- US-A- 5 919 189
- US-A- 5 919 189
- US-A- 6 033 400
- US-A- 6 033 400
- US-A1- 2014 088 592
- US-A1- 2014 088 592

## Beschreibung

Die Erfindung betrifft ein Hochfrequenzwerkzeug für medizinische Resektoskope zur Manipulation von Körpergewebe gemäß des Anspruchs 1.

Hochfrequenzwerkzeuge werden in der Medizin zur Manipulation von Körpergewebe wie beispielsweise dem Entfernen von Gewebe verwendet. Typische Anwendungen sind in der Urologie und dort beispielsweise in der Prostataresektion zu sehen. Dabei kann es sich bei dem Hochfrequenzwerkzeug um eine HF-Schneidelektrode handeln, die an einen Hochfrequenzgenerator angeschlossen wird, wobei der Generator mit einem Schalter von einem Operateur ein- und ausgeschaltet werden kann.

Bei angeschalteter Hochfrequenz schneidet eine an der HF-Schneidelektrode angeordnete Schneidschlinge sehr leicht und fast ohne Widerstand durch das zu entfernende Körpergewebe. Zum Schneiden des Gewebes wird ein Elektrodenträger des Resektoskopes mit dem Hochfrequenzwerkzeug bzw. der Schneidelektrode axial zum Resektoskop vor oder zurück bewegt. Somit wirken auf das Werkzeug bzw. auf die Schneidschlinge im Wesentlichen Kräfte parallel zur Achsrichtung des Resektoskopes.

Bei bekannten Schneidelektroden sind die beiden Enden der Schneidschlinge beispielsweise über Leitungsdrähte mit dem HF-Generator verbunden. Die Leitungsdrähte werden für die elektrische Verbindung mit dem Generator zunächst durch ein zwei Enden aufweisendes Gabelrohr geleitet, welches sodann zum Schlingenträger bzw. Elektrodenträger als Einzelrohr zusammenlaufen kann. Dieser Elektrodenträger erstreckt sich in Richtung des proximalen Endes des Resektoskopes, insbesondere zu einem Schlitten des Resektoskopes, wo die Leitungsdrähte auf bekannte Art und Weise mit dem Generator verbindbar sind. Beispiele sind im Stand der Technik in US 5 919 189 A, US 2014/088592 A1, US 6 033 400 A und DE 10 2005 042312 A1 offenbart.

Zur elektrischen Isolierung gegen den Elektrodenträger ist der Leitungsdraht bzw. sind die Leitungsdrähte mit einem Isolator, wie beispielsweise einem Isolierschlauch bzw. einem Kunststoffschlauch ummantelt. Die mit dem Isolator versehenen Bereiche der Schneidelektrode bzw. die Enden der Schneidschlinge werden zunächst durch jeweils ein Keramikröhrchen geleitet, bevor sie in den Elektrodenträger geführt werden. Diese Keramikröhrchen werden in bzw. auf eine Aufnahme an den Enden des Elektrodenträgers gesteckt. Die Keramikröhrchen dienen hauptsächlich dazu, die Temperatur, welche durch den HF-Generator an der Elektrode erzeugt wird, zu stabilisieren. Darüber hinaus stabilisieren die Keramikröhrchen die Elektrode mechanisch.

Insbesondere für Behandlungsprozeduren, wie beispielsweise bei der Enukleation erweist sich die vorbeschriebene Bauart für eine Elektrode bzw. Schneidelektrode als nachteilig, da die bei der Enukleation wirkenden Kräfte deutlich größer sind, im Vergleich zu Kräften, die bei einer Geweberesektion wirken. Bei den beispielhaft angeführten Behandlungsmethoden wirken auf die Elektrode nicht nur Kräfte in Achsrichtung des Resektoskopes, sondern auch quer dazu. Insbesondere durch diese Querkräfte können die Keramikröhrchen brechen. Neben einer dadurch verursachten mechanischen Instabilität der Elektrode kann es auch innerhalb der Elektrode bzw. des Elektrodenträgers zu einem Kurzschluss zwischen der Elektrode, den Leitungsdrähten und dem Elektrodenträger kommen. Somit ist die Benutzung der oben beschriebenen Schneidelektrode auf Anwendungen beschränkt, bei denen nur Kräfte in Achsrichtung des Resektoskopes auftreten und keine zusätzlichen oder nur geringe Querkräfte. Derzeit wird das Problem im Stand der Technik gelöst, indem ein Bügel zur Stabilisierung der Schneidschlinge vorgesehen ist, der die mechanischen Kräfte aufnimmt. Nachteilig dabei ist, dass der zusätzliche Bügel die Sicht und die Benutzung der Schneidklinge einschränkt.

Hiervon ausgehend, liegt der Erfindung die Aufgabe zugrunde, ein Hochfrequenzwerkzeug für ein medizinisches Resektoskop zu schaffen, das eine erhöhte Stabilität insbesondere gegenüber Querkräften aufweist.

Ein Hochfrequenzwerkzeug für medizinische Resektoskope zur Lösung der zuvor genannten Aufgabe weist die Merkmale des Anspruchs 1 auf. Demnach ist es erfindungsgemäß vorgesehen, dass die Elektrode zwei Enden aufweist, die zur Herstellung einer Verbindung mit dem Elektrodenträger jeweils in oder in distale Richtung gesehen vor einer Aufnahmeöffnung der Enden des Elektrodenträgers positionierbar sind und wobei die Elektrode zumindest in einem Bereich um eine Aufnahmeöffnung mit einem in dem Elektrodenträger geführten Leiterdraht, dessen Querschnitt geringer ist als der Querschnitt der Elektrode, elektrisch leitend kontaktiert ist. Dadurch, dass die beiden Enden des, insbesondere als bügelartige Schneidschlinge ausgebildetes, Hochfrequenzwerkzeuges bzw. der Elektrode direkt in die Aufnahmeöffnungen des Elektrodenträgers gesteckt werden, kann auf weitere stabilisierende Keramikröhrchen verzichtet werden. Somit weist das erfindungsgemäße Hochfrequenzwerkzeug eine erhöhte Stabilität gegenüber Querkräften auf. Die Enden des Hochfrequenzwerkzeuges können gegenüber dem Elektrodenträger elektrische Isoliert sein. Ebenso können der bzw. die mit dem Hochfrequenzwerkzeug verbundene Leiterdrähte gegenüber dem Elektrodenträger elektrisch isoliert sein. Der Elektrodenträger ist metallisch oder metallisiert oder als Isolator ausgebildet. Darüber hinaus kann es erfindungsgemäß vorgesehen sein, dass die Enden der Elektrode mit dem Leiterdraht vor den Aufnahmeöffnungen mittels einer Crimpzange verbunden sind. Der Querschnitt des Leiters der Elektrode kann zwischen 0,05 mm² und 1,8 mm², insbesondere 0,2 mm² bis 0,8 mm², vorzugsweise 0,4 mm², betragen. Durch den vergrößerten Querschnitt des Leiters der Elektrode lässt sich diese auf stabilere Art und Weise mit den Aufnahmen des Elektrodenträgers verbinden. Allein durch den erhöhten Querschnitt des Leiters der Elektrode weist diese schon eine erhöhte mechanische Stabilität gegenüber Querkräften auf.

Insbesondere sieht es die vorliegende Erfindung weiter vor, dass die Enden des Elektrodenträgers, insbesondere des Gabelrohres, zur Aufnahme der Enden der Elektrode, vorzugsweise der Schneidschlinge, aufgeweitet sind, insbesondere dass die Enden als Aufweitungen ausgebildet sind. Und zwar sind die Aufnahmen bzw. die Aufweitungen der Enden des Elektrodenträgers genauso ausgebildet, dass sie die Enden der Elektrode zusammen mit der die Enden der Elektrode ummantelnden Isolierung aufnehmen können. Die Öffnungen bzw. Aufweitungen am Elektrodenträger sind derart dimensioniert, dass die isolierten Enden der Elektrode nur ein geringes Spiel bzw. kein Spiel haben. Es ist insbesondere vorgesehen, dass die isolierten Enden der Elektrode in die Öffnungen des Elektrodenträgers gedrückt bzw. gepresst werden müssen. Durch diese Befestigung der Elektrode an dem Gabelrohr ist die Elektrode insbesondere durch Querkräfte belastbar, ohne dass die Elektrode mechanisch beschädigt wird.

Bevorzugt kann es außerdem vorgesehen sein, dass die Elektrode und der Leiterdraht einstückig aus einem elektrisch leitfähigen Material ausgebildet sind, wobei die Elektrode vorzugsweise ein Bereich des Leiterdrahtes mit einem vergrößerten Querschnitt darstellt. Durch diese Einstückigkeit lässt sich eine besonders große Stabilität erreichen, da die Elektrode und der Leiterdraht durch Einwirken äußerer Kräfte, insbesondere Querkräfte, nicht mehr ungewollt voneinander gelöst werden können.

Weiter kann es die vorliegende Erfindung vorsehen, dass der Leiterdraht durchgängig ausgebildet ist, insbesondere von einer Aufnahmeöffnung zu der anderen Aufnahmeöffnung führbar ist, und die Elektrode als Rohr zwischen den Enden des Elektrodenträgers über dem Leiterdraht geführt ist. Dabei ist ein Innendurchmesser des Rohres so zu wählen, dass vorzugsweise ein ständiger, leitender Kontakt zwischen dem Rohr und dem Leiterdraht beseht. Da das Rohr somit über den Leiterdraht geführt ist, kann eine erhöhte Stabilität erreicht werden, da das Rohr untrennbar mit dem Leiterdraht verbunden ist.

Insbesondere kann es erfindungsgemäß denkbar sein, dass die Enden der Elektrode (13) mit einem Konstruktionswerkstoff ummantelt sind, dessen Festigkeit größer als 80 MPa ist. Ein weiteres vorteilhaftes Ausführungsbeispiel der vorliegenden Erfindung kann es vorsehen, dass die Enden der Elektrode von einem Isolator, insbesondere einem Kunststoffschlauch, vorzugsweise einem PTFE-Schlauch, ummantelt sind. Darüber hinaus ist es denkbar, dass der Schlauch aus einem beliebigen, isolierenden Material ausgebildet ist, das sich dichtanliegend um die Elektrode legen lässt. Die Wandstärke des Isolierschlauches kann 0,1 mm bis 0,5 mm, insbesondere 0,1 mm bis 0,3 mm, vorzugsweise 0,2 mm betragen.

Ein weiteres besonders bevorzugtes Ausführungsbeispiel der vorliegenden Erfindung kann es vorsehen, dass die Enden der Elektrode jeweils durch PEEK-Röhrchen oder PPS-Röhrchen von den Enden des Elektrodenträgers elektrisch isoliert sind. Diese PEEK-Röhrchen können alternativ oder zusätzlich zum Isolierschlauch die Enden der Elektrode gegen die Enden des Elektrodenträgers isolieren. Durch die PEEK-Röhrchen wird die Elektrode weiter stabilisiert, da PEEK eine große mechanische Festigkeit, insbesondere gegenüber Querkräften, aufweist.

Die vorliegende Erfindung kann es des Weiteren bevorzugt vorsehen, dass die Enden der Elektrode mit den Leiterdrähten mechanisch fest verbunden, insbesondere verschweißt, verlötet, verklemmt, verquetscht oder dergleichen sind. Es ist darüber hinaus denkbar, dass die Enden der Elektrode und die Leitdrähte mittels einer Crimpzange miteinander verbunden werden. Durch diese feste Verbindung der Elektrode mit den Leiterdrähten ist die Elektrode insbesondere gegenüber Kräften in Achsrichtung stabil. Darüber hinaus kann die Elektrode durch die Leiterdrähte in die Aufnahmen bzw. Aufweitungen des Elektrodenträgers gezogen werden, wodurch eine weitere Stabilisierung der Elektrode erzielt werden kann.

Insbesondere sieht es die vorliegende Erfindung weiter vor, dass die Isolierung der Enden der Elektrode und der Drähte einstückig ist, sich vorzugsweise aus mehreren Stücken zusammensetzt. Der Vorteil einer einstückigen Isolierung besteht darin, dass eine durchgehende Isolierung ohne Unterbrechung besteht. Durch die unvermeidbaren Unterbrechungen einer mehrstückigen Isolierung könnten beispielsweise Flüssigkeiten wie Salzlösungen oder dergleichen an die Leiter herantreten und zu einem Kurzschluss führen. Ein Vorteil einer mehrstückigen Isolierung kann darin gesehen werden, dass für verschiedene Leiterabschnitte verschiedene Isolatoren bzw. verschiedene Isolatorstärken verwendet werden könnten. Bereiche, die einer erhöhten mechanischen Belastung ausgesetzt sind, können beispielsweise mit einem dickeren Isolierschlauch versehen sein. So kann es beispielsweise vorgesehen sein, dass sowohl die Enden der Elektrode mit einem Isolierschlauch versehen sind, als auch die einzelnen Leitungsdrähte. Alternativ ist es denkbar, dass die Enden der Elektrode und der jeweilige Leitungsdraht durch einen einzigen Isolierschlauch gegenüber den Wandungen des Elektrodenträgers bzw. des Gabelrohres elektrisch isoliert sind.

Weiter kann es die Erfindung vorsehen, dass die von der Isolierung ummantelten Enden der Elektrode in den Aufweitungen des Elektrodenträgers fest verbunden, insbesondere verquetscht, geklebt oder dergleichen sind. Durch diese formfeste Verbindung der Elektrode mit dem Elektrodenträger bzw. dem Gabelrohr kann die erhöhte und geforderte Stabilität der Elektrode bzgl. Querkräfte erreicht werden.

Ein weiteres Ausführungsbeispiel der vorliegenden Erfindung kann es vorsehen, dass die Enden der Elektrode zur Stabilisierung durch Röhrchen aus Stahl steckbar sind, die direkt vor den Öffnungen des Elektrodenträgers angeordnet sind. Dabei kann es denkbar sein, dass die Metallröhrchen integraler Bestandteil der Elektrode oder der Enden des Elektrodenträgers sind. Die Isolierung der Elektrode ist dann wieder über die zusätzlichen Röhrchen führbar, oder durch die Röhrchen hindurch. So ist es denkbar, dass isolierte Röhrchen als integraler Bestandteil der Elektrode in Aufweitungen des Elektrodenträgers steckbar sind. Dadurch könnte die Kontaktfläche zwischen der Elektrode und der Aufnahme des Elektrodenträgers vergrößert werden. Durch eine Vergrößerung der Kontaktfläche können die auf die Elektrode wirkende Kräfte besser verteilt bzw. kompensiert werden, was zu einer erhöhten Stabilität führt.

Ein bevorzugtes Ausführungsbeispiel eines Hochfrequenzwerkzeuges wird im Folgenden Anhand der Zeichnung näher beschrieben. In dieser zeigen:
- Fig. 1: ein HF-Resektoskop mit einem Hochfrequenzwerkzeug,
- Fig. 2: eine Sicht auf einen distalen Bereich einer Elektrode, und
- Fig. 3: einen Schnitt durch die Elektrode gemäß der Fig. 2.

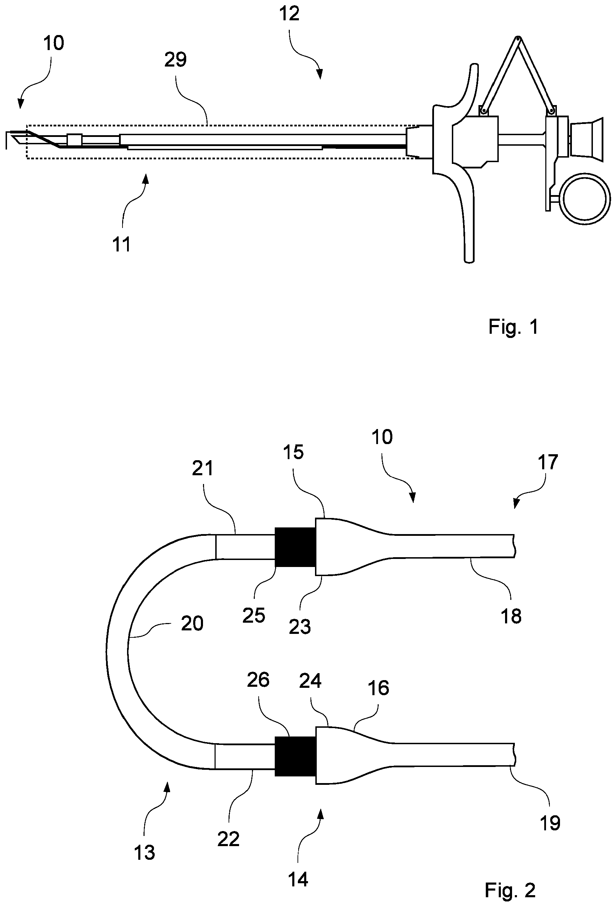

Das erfindungsgemäße Hochfrequenzwerkzeug 10 ist an einem distalen Ende eines Schaftes 11 eines Resektoskopes 12 angeordnet. In der Fig. 1 ist exemplarisch ein HF-Resektoskop 12 bzw. ein Transporteur eines HF-Resektoskopes 12 mit einem angedeuteten äußeren Schaft 29 dargestellt. Das Hochfrequenzwerkzeug 10 befindet sich in der Fig. 1 in einer aus dem äußeren Schaft 29 herausgeschobenen Arbeitsstellung. Das in den Fig. 1, 2 und 3 dargestellte Hochfrequenzwerkzeug 10 ist beispielhaft als eine eine Schneidschlinge aufweisende Schneidelektrode ausgebildet. Es sei an dieser Stelle jedoch ausdrücklich darauf hingewiesen, dass die vorliegende Erfindung nicht auf das hier dargestellte Ausführungsbeispiel eines Resektoskopes 12 bzw. einer Schneidelektrode eingeschränkt ist. Vielmehr kann das Hochfrequenzwerkzeug 10 gemäß der Erfindung für jedes medizinische Instrument verwendet werden.

Da der Gegenstand der vorliegenden Erfindung in dem Hochfrequenzwerkzeug 10 zu sehen ist, soll auf eine weitere Beschreibung des dargestellten Resektoskopes 12 verzichtet werden. Für eine detaillierte Beschreibung wird auf den umfänglichen Stand der Technik verwiesen.

In der Fig. 2 ist eine Ausschnittsvergrößerung des erfindungsgemäßen Hochfrequenzwerkzeuges 10 dargestellt. Im Wesentlichen besteht dieses Hochfrequenzwerkzeug 10 aus einer bügelartigen Elektrode 13 und dem Elektrodenträger 14. Von dem Elektrodenträger 14 sind in der Fig. 2 lediglich zwei Enden 15, 16 dargestellt. Der Elektrodenträger 14 wird durch ein ebenfalls nur teilweise gezeigtes Gabelrohr 17 dargestellt. Das Gabelrohr 17 weist zwei rohrartige Arme 18, 19 auf, die zu einem Rohr zusammenlaufen; zusammen formen sie den Elektrodenträger 14.

Die hier dargestellte Elektrode 13 weist einen Bogen 20 oder Bügel auf, der sich nach unten in die Zeichenebene erstreckt. Es ist jedoch auch denkbar, dass dieser Bogen 20 als einfache Verbindung der Enden 21 und 22 ausgebildet ist und in der gleichen Ebene wie diese liegt. Durch die Gestaltung des Bogens 20 lässt sich die Schnitttiefe bzw. das Schnittmuster der Elektrode 13 bestimmen.

Zur Befestigung der Elektrode 13 an dem Gabelrohr 17 bzw. an dem Elektrodenträger 14 werden die Enden 21, 22 in jeweils eine Öffnung bzw. Aufweitung 23, 24 an den Enden 15, 16 des Elektrodenträgers 14 gesteckt. Da sowohl die Elektrode 13 als auch die Arme 18, 19 des Gabelrohres 17 bzw. des Elektrodenträgers 14 metallisch sein können, ist die ebenfalls metallische Elektrode 13 bzw. sind die Enden 21, 22 der Elektrode 13 gegen die Aufweitungen 23, 24 zu isolieren. Dazu sieht es die Erfindung vor, sowohl das Ende 21 als auch das Ende 22 mit einem Isolierschlauch 25, 26 zu ummanteln. Bei diesem Isolierschlauch 25, 26 kann es sich beispielsweise um einen Kunststoffschlauch bzw. um einen PTFE-Schlauch handeln. Die Wandstärke dieses Schlauches kann 0,1 mm bis 0,5 mm bzw. 0,2 mm bis 0,4 mm, insbesondere 0,3 mm betragen, wobei die Wandstärke vor allem vom Material und der geforderten Durschlagfestigkeit anhängig ist.

Die Enden 21, 22 der Elektrode 14 sind jeweils mit einem Leiterdraht 27, 28 fest verbunden. Bei dieser festen Verbindung zwischen den Enden 21, 22 und den Leiterdrähten 27, 28 kann es sich um eine Quetsch-, Löt-, Steckverbindung oder dergleichen handeln.

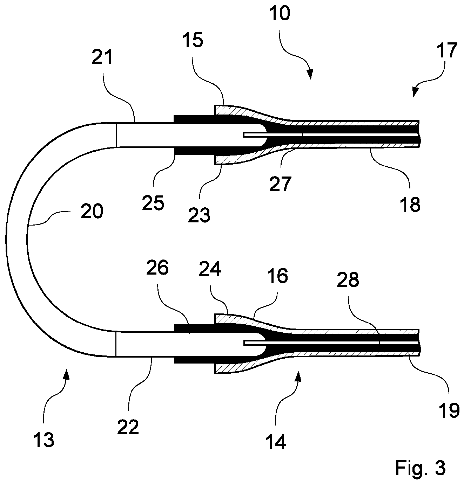

Genau wie die Enden 21, 22 sind auch die Leiterdrähte 27, 28 durch eine Isolierung gegen die Wandungen der Arme 18, 19 elektrisch isoliert. Bei dieser Isolierung kann es sich, wie in der Fig. 3 dargestellt, um die gleichen Isolierschläuche 25, 26 handeln, die auch die Enden 21, 22 elektrisch isolieren. Die Isolierschläuche 25, 26 laufen dann zu einem Isolierschlauch zusammen.

Das Hochfrequenzwerkzeug 10 wird mit seinen Enden 21, 22 derart in die Aufweitungen 23, 24 gesteckt, dass die Enden 21, 22 zusammen mit den Isolierschläuchen 25, 26 eine nahezu feste Verbindung mit den Aufweitungen 23, 24 ausbildet. Durch diese Steckverbindung zwischen der Elektrode 13 und den Leiterdrähten 27, 28 ist das erfindungsgemäße Hochfrequenzwerkzeug 10 stabil gegenüber Querkräften. Das heißt, dass bei einer Kraftausübung quer zur Längsachse des Resektoskopes 12, insbesondere die Aufweitungen 23, 24 des Elektrodenträgers 17 und die Elektrode 13 mechanisch nicht beschädigt werden.

Es ist durchaus denkbar und im Rahmen der Erfindung vorgesehen, dass die in den Fig. 2 und 3 dargestellte Formen der Arme 18, 19 bzw. der Aufweitungen 23, 24 des Elektrodenträgers 17 anders ausgestaltet ist. Darüber hinaus ist es erfindungsgemäß vorgesehen, dass anstatt der Isolierschläuche 25, 26 oder zusätzlich zu den Isolierschläuchen 25, 26 die Enden 21, 22 der Elektrode 14 durch weitere oder alternative Isolatoren, wie beispielsweise PEEK-Röhrchen, gegenüber dem Elektrodenträger 17 isoliert werden.

### Bezugszeichenliste

- 10: Hochfrequenzwerkzeug
- 11: Schaft
- 12: Resektoskop
- 13: Elektrode
- 14: Elektrodenträger
- 15: Ende
- 16: Ende
- 17: Gabelrohr
- 18: Arm
- 19: Arm
- 20: Bogen
- 21: Ende
- 22: Ende
- 23: Aufweitung
- 24: Aufweitung
- 25: Isolierschlauch
- 26: Isolierschlauch
- 27: Leiterdraht
- 28: Leiterdraht
- 29: Äußerer Schaft

## Patentansprüche

1. Hochfrequenzwerkzeug (10), insbesondere Schneidelektrode, für medizinische Resektoskope (12) zur Manipulation von Körpergewebe mit einem in Achsrichtung des Resektoskopes (12) bewegbaren Elektrodenträger (14), an dessen distalen, gabelförmigen Enden (15, 16) eine mit Hochfrequenzstrom beaufschlagbare Elektrode (13) gehaltert ist, wobei die Elektrode (13) zwei Enden (21, 22) aufweist, die zur Herstellung einer Verbindung mit dem Elektrodenträger (14) jeweils in oder in distale Richtung gesehen vor einer Aufnahmeöffnung der Enden (15, 16) des Elektrodenträgers (14) positioniert sind und wobei die Elektrode (13) zumindest in einem Bereich um eine Aufnahmeöffnung mit einem in dem Elektrodenträger (14) geführten Leiterdraht (27, 28), dessen Querschnitt geringer ist als der Querschnitt der Elektrode (13), elektrisch leitend kontaktiert ist und wobei die Enden (15, 16) des Elektrodenträgers (14) zur Aufnahme der Enden (21, 22) der Elektrode (13) als Aufweitungen (23, 24) ausgebildet sind,
**dadurch gekennzeichnet, dass**
die Elektrode (13) und der Leiterdraht (27, 28) einstückig aus einem elektrisch leitfähigem Material ausgebildet sind, wobei die Elektrode (13) ein Bereich des Leiterdrahtes (27, 28) mit einem vergrößertem Querschnitt darstellt, und wobei der Leiterdraht (27, 28) durchgängig ausgebildet ist und die Elektrode (13) als Rohr zwischen den Enden (15, 16) des Elektrodenträgers (14) über dem Leiterdraht (27, 28) geführt ist.

2. Hochfrequenzwerkzeug (10) nach Anspruch 1, **dadurch gekennzeichnet, dass** die Enden der Elektrode (13) mit einem Konstruktionswerkstoff ummantelt ist, dessen Festigkeit größer als 80 MPa ist.

3. Hochfrequenzwerkzeug (10) nach einem der vorangegangenen Ansprüche, **dadurch gekennzeichnet, dass** die Enden (21, 22) der Elektrode (13) und/oder die Leiterdrähte (27, 28) von einem Isolator, vorzugsweise einem Isolatorschlauch (25, 26), insbesondere einem Kunststoffschlauch ummantelt sind.

4. Hochfrequenzwerkzeug (10) nach einem der vorangegangenen Ansprüche, **dadurch gekennzeichnet, dass** die Enden (21, 22) der Elektrode (13) jeweils durch PEEK-Röhrchen oder PPS-Röhrchen von den Enden (15, 16) des Elektrodenträgers (14) elektrisch isoliert sind.

5. Hochfrequenzwerkzeug (10) nach einem der vorangegangenen Ansprüche, **dadurch gekennzeichnet, dass** die Isolierung der Enden (21, 22) der Elektrode (13) und der Leitungsdrähte (27, 28) einstückig ausgebildet ist, sich vorzugsweise aus mehreren Stücken zusammensetzt.

6. Hochfrequenzwerkzeug (10) nach einem der vorangegangenen Ansprüche, **dadurch gekennzeichnet, dass** die von der Isolierung ummantelten Enden (21, 22) der Elektrode (13) in den Aufweitungen (23, 24) des Elektrodenträgers (14) fest verbunden, insbesondere verquetscht, geklebt oder dergleichen, sind.

7. Hochfrequenzwerkzeug (10) nach einem der vorangegangenen Ansprüche, **dadurch gekennzeichnet, dass** die Enden (21, 22) der Elektrode (13) zur Stabilisierung durch Röhrchen aus Stahl steckbar sind, die direkt vor den Aufnahmeöffnungen des Elektrodenträgers (14) angeordnet sind.

8. Hochfrequenzwerkzeug (10) nach Anspruch 7, **dadurch gekennzeichnet, dass** die Röhrchen integraler Bestandteil der Elektrode (13) oder der Enden (15, 16) des Elektrodenträgers (14) sind.

## Claims

1. Radiofrequency tool (10), in particular cutting electrode, for medical resectoscopes (12) for manipulating body tissue using an electrode carrier (14) which is moveable in the axial direction of the resectoscope (12) and on the distal, fork-shaped ends (15, 16) of which is secured an electrode (13) to which radiofrequency current can be applied, wherein the electrode (13) has two ends (21, 22) which in order to establish a connection to the electrode carrier (14) are each positioned in or, as viewed in the distal direction, in front of a receiving opening of the ends (15, 16) of the electrode carrier (14), and wherein, at least in an area around a receiving opening, the electrode (13) is in electrically conductive contact with a conductor wire (27, 28) which is guided in the electrode carrier (14) and whose cross section is smaller than the cross section of the electrode (13), and wherein the ends (15, 16) of the electrode carrier (14) are formed as expansions (23, 24) in order to receive the ends (21, 22) of the electrode (13),
**characterized in that**
the electrode (13) and the conductor wire (27, 28) are formed in one piece from an electrically conductive material, wherein the electrode (13) represents a region of the conductor wire (27, 28) with an increased cross section, and wherein the conductor wire (27, 28) is formed continuously, and the electrode (13) is guided as a tube between the ends (15, 16) of the electrode carrier (14) over the conductor wire (27, 28).

2. Radiofrequency tool (10) according to Claim 1, **characterized in that** the ends of the electrode (13) are covered by a construction material whose strength is greater than 80 MPa.

3. Radiofrequency tool (10) according to either of the preceding claims, **characterized in that** the ends (21, 22) of the electrode (13) and/or the conductor wires (27, 28) are covered by an insulator, preferably an insulator tube (25, 26), in particular a plastic tube.

4. Radiofrequency tool (10) according to any of the preceding claims, **characterized in that** the ends (21, 22) of the electrode (13) are each electrically insulated from the ends (15, 16) of the electrode carrier (14) by PEEK tubes or PPS tubes.

5. Radiofrequency tool (10) according to any of the preceding claims, **characterized in that** the insulation of the ends (21, 22) of the electrode (13) and of the conductor wires (27, 28) is formed in one piece, preferably composed of a plurality of pieces.

6. Radiofrequency tool (10) according to any of the preceding claims, **characterized in that** the ends (21, 22) of the electrode (13) covered by the insulation are securely connected, in particular crimped, adhesively bonded or the like, in the expansions (23, 24) of the electrode carrier (14).

7. Radiofrequency tool (10) according to any of the preceding claims, **characterized in that** for stabilization purposes, the ends (21, 22) of the electrode (13) can be passed through steel tubes which are arranged directly in front of the receiving openings of the electrode carrier (14).

8. Radiofrequency tool (10) according to Claim 7, **characterized in that** the tubes are an integral part of the electrode (13) or of the ends (15, 16) of the electrode carrier (14).

## Revendications

1. Outil à haute fréquence (10), notamment électrode de coupe, pour des résectoscopes médicaux (12) destiné à la manipulation de tissus corporels, comprenant un porte-électrode (14) mobile dans la direction axiale du résectoscope (12), aux extrémités distales en forme de fourche duquel (15, 16) est maintenue une électrode (13) pouvant être soumise à un courant à haute fréquence, l'électrode (13) possédant deux extrémités (21, 22) qui, pour l'établissement d'une liaison avec le porte-électrode (14), sont respectivement situées dans ou, vues dans la direction distale, devant une ouverture de réception des extrémités (15, 16) du porte-électrode (14) et l'électrode (13) étant mise en en contact de manière électriquement conductrice, au moins dans une zone autour d'une ouverture de réception, avec un fil conducteur (27, 28) guidé dans le porte-électrode (14), dont la section transversale est inférieure à la section transversale de l'électrode (13), et les extrémités (15, 16) du porte-électrode (14) étant réalisées sous la forme d'élargissements (23, 24) servant à recevoir les extrémités (21, 22) de l'électrode (13),
**caractérisé en ce que**
l'électrode (13) et le fil conducteur (27, 28) sont réalisés d'une seule pièce en un matériau électriquement conducteur, l'électrode (13) représentant une zone du fil conducteur (27, 28) avec une section transversale agrandie, et le fil conducteur (27, 28) étant réalisé de manière continue et l'électrode (13) étant guidée sous forme de tube entre les extrémités (15, 16) du porte-électrode (14) au-dessus du fil conducteur (27, 28).

2. Outil à haute fréquence (10) selon la revendication 1, **caractérisé en ce que** les extrémités de l'électrode (13) sont enrobées d'un matériau de construction dont la résistance est supérieure à 80 MPa.

3. Outil à haute fréquence (10) selon l'une des revendications précédentes, **caractérisé en ce que** les extrémités (21, 22) de l'électrode (13) et/ou les fils conducteurs (27, 28) sont enrobés d'un isolant, de préférence d'une gaine isolante (25, 26), notamment d'une gaine en matière plastique.

4. Outil à haute fréquence (10) selon l'une des revendications précédentes, **caractérisé en ce que** les extrémités (21, 22) de l'électrode (13) sont respectivement isolées électriquement des extrémités (15, 16) du porte-électrode (14) par des petits tubes en PEEK ou des petits tubes en PPS.

5. Outil à haute fréquence (10) selon l'une des revendications précédentes, **caractérisé en ce que** l'isolation des extrémités (21, 22) de l'électrode (13) et des fils conducteurs (27, 28) est réalisée d'une seule pièce, se compose de préférence de plusieurs pièces.

6. Outil à haute fréquence (10) selon l'une des revendications précédentes, **caractérisé en ce que** les extrémités (21, 22) de l'électrode (13) enrobées par l'isolant sont reliées à demeure, notamment par sertissage, collage ou similaire, dans les élargissements (23, 24) du porte-électrode (14).

7. Outil à haute fréquence (10) selon l'une des revendications précédentes, **caractérisé en ce que** les extrémités (21, 22) de l'électrode (13) peuvent être enfichées, en vue de la stabilisation, à travers des petits tubes en acier qui sont disposés directement devant les ouvertures de réception du porte-électrode (14).

8. Outil à haute fréquence (10) selon la revendication 7,
**caractérisé en ce que** les petits tubes font partie intégrante de l'électrode (13) ou des extrémités (15, 16) du porte-électrode (14).
